(19) Europäisches Patentamt
European Patent Office
Office européen des brevets

(11) Publication number: **0 077 078**
**B1**

(12) # EUROPEAN PATENT SPECIFICATION

(45) Date of publication of patent specification: **15.10.86**

(51) Int. Cl.⁴: **A 01 N 43/36,** A 01 N 43/40, A 01 N 43/46, A 01 N 25/02

(21) Application number: **82109477.8**

(22) Date of filing: **13.10.82**

(54) **Method for improved plant pest control.**

(30) Priority: **13.10.81 US 310948**

(43) Date of publication of application: **20.04.83 Bulletin 83/16**

(45) Publication of the grant of the patent: **15.10.86 Bulletin 86/42**

(84) Designated Contracting States: **BE CH DE FR GB IT LI NL SE**

(56) References cited:
**EP-A-0 056 261**
**GB-A-1 553 309**
**GB-A-1 553 310**
**GB-A-2 029 832**
**US-A-3 988 318**
**US-A-3 988 351**

(73) Proprietor: **NELSON RESEARCH & DEVELOPMENT COMPANY**
**19712 MacArthur Boulevard**
**Irvine, CA 92715 (US)**

(72) Inventor: **Rajadhyaksha, Vithal J.**
**27435 Esquina**
**Mission Viejo CA 22691 (US)**

(74) Representative: **Kraus, Walter, Dr. et al**
**Patentanwälte Kraus, Weisert & Partner**
**Thomas-Wimmer-Ring 15**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 077 078 B1

**Description**

BACKGROUND OF THE INVENTION

Field of the Invention

The present invention generally relates to a method of treatment of plants. More particularly, the invention relates to an improved method of plant pest control

Background of the Prior Art

Pesticides are chemicals designed to combat the attacks of various pests on agricultural and horticultural crops. They fall into three major classes: insecticides, fungicides and herbicides (or weed killers). There are also rodenticides (for control of vertebrate pests), nematicides (to kill microscopic eelworms), molluscicides (to kill slugs and snails) and acaracides (to kill mites).

Pesticides may also be divided into two main types, namely contact or nonsystemic pesticides and systemic pesticides. Contact or surface pesticides do no appreciably penetrate plant tissues and are consequently not transported or translocated, within the plant vascular system. The earlier insecticides, fungicides and herbicides were of this type; their disadvantages are that they are susceptible to the effects of weathering (wind, rain and sunlight) over long periods and new plant growth will be left unprotected and hence open to attack by insect and fungal pests. The early agricultural fungicides were, therefore, protectant fungicides—in other words they are designed to prevent the development of the fungal spores, but once the fungus has become established and infection starts to ramify through the plant tissue such nonsystemic fungicides possess little eradicant action and usually cannot halt the infection.

In contrast, many of the more recent pesticides are systemic in character—these can effectively penetrate the plant cuticle and move through the plant vascular system. Examples are provided by the phenoxyacetic acid selective herbicides, certain organophosphorus insecticides and the more recently discovered systemic fungicides like benomyl.

Systemic fungicides are also sometimes termed plant chemotherapeutants and can not only protect the plant from fungal attack, but also cure or inhibit an established infection. They are little affected by weathering and will also confer immunity on all new plant growth.

Pests can be divided into various groups. In the plant kingdom, characterized by the ability of the organism to photosynthesize carbohydrates from air and water with the aid of the green pigment chlorophyll, higher plants growing where man does not want them are termed weeds and are important pests. Of the lower plants, algae are not generally of as great importance as pests, although in some circumstances, e.g., in lakes and other slow moving water, excessive algal growth or "bloom" may cause considerable damage and require treatment with chemicals (algicides).

Fungi or nonphotosynthetic plants cannot obtain their nutrients from air and water since they do not have chlorophyll; consequently they feed directly on decaying plant or amimal matter (saprophytic fungi) or on living plants or animals (parasitic fungi). There are thousands of different species of fungi mainly found in soil—some, like yeasts, are unicellular while others are composed of a network of branched filaments (hyphae). A number of fungi are serious pests attacking both living crop plants and also crops in storage.

Several bacteria are causal agents of plant diseases, although they are not nearly as important as the phytopathogenic fungi. Bacteria can be observed under the microscope and can be classified according to their shape; thus a spherical bacterium is termed a coccus while a rod-shaped one is a bacillus.

Viruses, like bacteria and fungi, attack plants and animals and some species cause significant plant diseases. Viruses form a distinct category of living organism because the are not true cells. Unlike bacteria they are too small (10—30 nm) in diameter to be observed with an ordinary microscope, but they can be revealed under the electron microsope—each virus consists of a single strand of DNA or RNA surrounded by a protective coat of protein.

Several higher animals (vertebrates) are important pests, e.g., mice, rats and rabbits; another group of pests is represented by the true insects (arthropods) which are invertebrates. The latter possess three pairs of legs and the adult body has three parts; the arachnids (mites and ticks) differ from true insects in having no distinct division of the body into three parts; also they usually have four pairs of legs. In the lower orders of animals, certain nematodes, parasitic worms often with unsegmented bodies, are important crop pests.

If pesticides are to be active they must reach the ultimate site of action within the target organism. Thus even surface fungicides, like Bordeaux mixture, must be able to penetrate the fungal spore; similarly contact insecticides have to penetrate the insect cuticle, and contact herbicides the plant cuticle when they impinge on it. The requirements if the pesticides are to be systemic in action are much more stringent because in addition they must have the capacity to be absorbed by the roots or leaves or seeds of plants and be delivered to other parts of the plant. In this way the whole plant, including new growth, is protected from fungal attack, or rendered poisonous to any insect that eats or sucks it.

In US—A—3,988,318 a low molecular weight complex of a lactam and a hydroxy phenolic compound is described. This complex is a 1:1 complex. There is not used a delivery enhancing amount of the substituted lactam but it is used a 1:1 complex of lactam as pesticide. To form these complexes with lactams the pesticides must have a phenolic hydroxyl group or in general a strong acid group, not all pesticides fall into this category.

In GB—A—1,553,309 there is described a composition comprising a physiologically active agent and a lactam compound. The method for improved plant pest control as claimed in this invention is not known from this citation. It is known that the physiological and membrane differences between animals having a stratum corneum and plants and various plant pests exist. In animals the epidermis of the skin has an exterial layer of dead cells called the stratum corneum which is tightly compacted and oily.

By contrast, the exoskeleton of an insect comprises "a semi-rigid accellular cuticle that covers all external parts, the structural components of the cuticle are protein and chitin, a complex of polyacetylglucosamine". It is common knowledge that arachnids have a comparable exoskeleton of chitin. On the othe hand, nematodes are covered by a non-cellular elastic cuticle chiefly composed of sclero· proteins (not chitin). The penetration through all these different membranes differs.

In GB—A—2,029,832 there is utilized N-methyl-2-pyrrolidone with a plant growth regulator, chloroethyl phosphonic acid, in a 1:1 ratio similar to U.S. patent 3,988,318. N-methyl-2-pyrrolidone is water-soluble and has different permeation properties than 1-Dodecylazacycloheptan-2-one and the other lactams described in this application.

## SUMMARY OF THE INVENTION

We have now discovered an improved method of plant pest control by enhancing the delivery of pesticides to plant pests. The foregoing is accomplished through the use of a compound which, when combined with a pesticide, enhances the delivery of the pesticide to plant pests. The composition containing the delivery-enhancing compound and pesticide may be applied directly to the plant pest by topical application or indirectly by topical application to the plants to be protected. The latter indirect method of application enables the pesticide to reach its ultimate site of action, namely the plant pest, after the plant pest has come in contact with the treated plant.

The invention, therefore, relates to an improved method of plant pest control comprising contacting a plant or plant pest with a composition comprising an effective amount of a plant pesticide and 0.1 to 10% by weight of the pesticide composition of a compound having the structural formula:

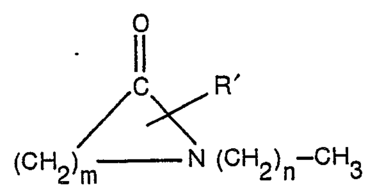

wherein R′ is H or alkyl having 1—4 carbon atoms, m is 5-7 and n is 0—11.

This invention relates also to a pesticide composition comprising a surfactant suitable for use as a herbicide adjuvant and 0.1 to 10% by weight of the pesticide composition of a compound having the structural formula

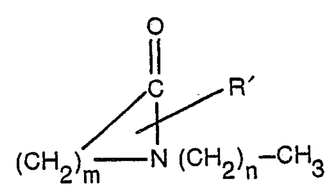

wherein R′ is H or alkyl having 1—4 carbon atoms, m is 5—7 and n 0—11.

This invention relates also to a composition comprising an effective amount of a plant pesticide and 0.1 to 10% by weight of the pesticide composition of a compound having the structural formula

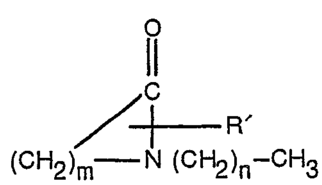

wherein R′ is H or alkyl having 1—4 carbon atoms, m is 5—7 and n is 0—11.

In one preferred embodiment R′ is H. The preferred compound is 1-n-dodecylazacycloheptan-2-one.

## DETAILED DESCRIPTION OF THE INVENTION

The 1-substituted azacycloalkan-2-ones used in this invention are made by methods described below and as further described in the Examples. Typical examples of compound included in the foregoing formula are the following:

1-methylazacycloheptan-2-one
1-n-propylazacycloheptan-2-one
1-n-butylazacycloheptan-2-one
1-n-pentylazacycloheptan-2-one
1-n-hexylazacycloheptan-2-one
1-n-heptylazacycloheptan-2-one
1-n-octylazacycloheptan-2-one
1-n-nonylazacycloheptan-2-one
1-n-decylazacycloheptan-2-one
1-n-dodecylazacycloheptan-2-one
1-n-butylazacyclononan-2-one
1-n-octylazacyclononan-2-one

The compounds covered by the general formula may be prepared by treating an azacycloalkan-2-one with an alkyl or aralkyl halide or mesylate in the presence of a base, e.g., sodium hydride. The reaction is carried out under anhydrous conditions in a hydrocarbon solvent, for example, dry toluene at reflux temperature for about 10 to 72 hours in an inert atmosphere, for example, nitrogen. This method is outlined below:

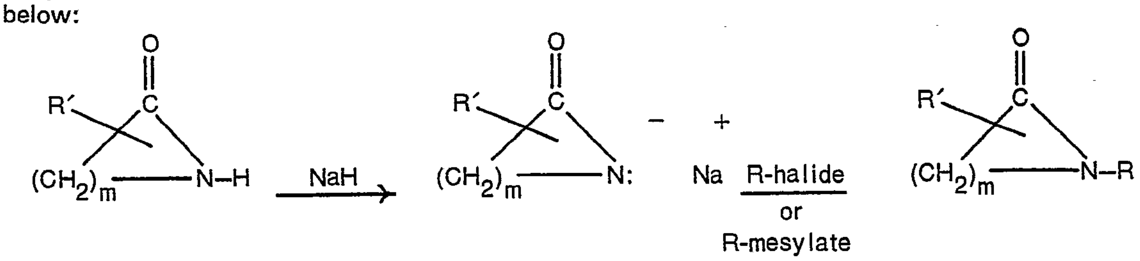


Alternatively, a lactone of an alkanoic acid may be heated with an alkyl, aryl or alkyl amine (with or without solvent) for about 20 to 72 hours at about 180—250°C with removal of water to obtain the corresponding 1-substituted azacycloalkan-2-one shown below:

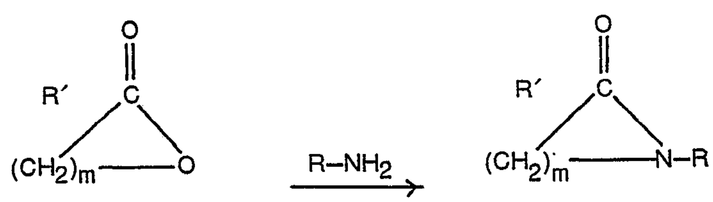


Typical plant pesticides include conventional pesticides, including, but not limited to, insecticides, fungicides, herbicides, rodenticides, nematicides, molluscicides, acaricides, plant growth regulators, fumigants, anti-feeding compounds, chemosterilants, hormones and growth inhibitors.

Suitable pesticides include botanical insecticides such as, for example, nicotine, derris (rotenone) and pyrethrum; synthetic insecticides including dinitrophenols, such as, for example, DNOC; organic thiocyanates such as, for example, lethane and thanite; organochlorine insecticides including DDT and related compounds; hexachlorocyclohexane; insecticides containing the cyclodiene group such as, for example, aldrin and dieldren; organophosphorous insecticides including malathion, mevinphos, rogar, dimethoate, nenozan, miral, diazinon, dursbon, bayrusil; organocarbonate insecticides including pirimicarb, carbaryl, baygon, propoxur, zectron, carbofuran, aldicarb (Temik), methomul (Lonnate); fungicides including phenylmercury compounds, naban, metham sodium, thiron; compounds containing the n-trichloromethylthio group, such as, for example, captan, folpet and oifolatan; dinitrophenols, including dinocap (Karathane); chlorobenzynes and related compounds, quinones such as, for example, dodine and roural, sulphonamides; benzimidozoles; thiophonates; oxathiins; pyrimadines; piperozine, morpholine and azepine derivatives; organophosphorous compounds including wepsyn, kitazin and conen, herbicides including carboxylic acid herbicides, such as, for example, 2,4-D MCPA, 2,3,6-TBA, IAA, picloram and dichlobenil; chloroaliphatic acids such as dalapan and TCA, and heterocyclic compounds such as atrozine (Gesaprim); triazale such as amitrole, pyrazon, bromacil, endothal; bipyridinum herbicides including paraquat and diquat; benzonitriles; diphenyl ethers; organophosphorous compounds such as, for example, phosphorothiolates such as bensulide; phosphoramidates such DMPA (Zytron); phosphonates such as glyphosate; plant growth regulators; fumigants; rodenticides including anticoagulants such as warfarin, pidone and norbormide (Raticote); sleep including narcotic drugs such as

chloralose; gophacide, silatrane and crimidine; nematicides such as dazomet and nellite; molluscicides such as metaldehyd, methiocasb and frescon; repellants, antifeeding compounds such as ZIP; chemosterilants, hormones and growth inhibitors. Further examples of pesticides suitable for use in the present invention are known in the art (see, for example, R. Cremlyn, *Pesticides, Preparation and mode of action,* John Wiley and Sons, 1979; F. McEwan et al., *The Use and Significance of Pesticides in the Environment,* John Wiley and Sons, 1979; D. Roberts, *Fundamentals of Plant-Pest Control,* E. H. Freeman and Company, 1978.

The method of application of the pesticide composition described herein is conventional. See, for example, G. Hartley et al., *Chemicals for Pest Control,* Chapter 15, "Application of Pesticides", Pergamon Press, 1969.

Adjuvants, such as surfactants, may also be used in the present invention. Suitable surfactants include, but are not limited to, the following:

| Trade Name | Chemical composition | Ionogenic class |
|---|---|---|
| Aerosol OT | sodium dioctyl sulfosuccinate | anionic |
| AHCO DD 50 | alkybenzyl quaternary ammonium halide | cationic |
| Alrowet D—65 | sodium dioctyl sulfosuccinate | aniomic |
| Aqua Gro | 50% polyoxyethylene ester; 50% polyoxyethylene ether | nonionic |
| Atlas NNO | glycerol mannitan laurate | nonionic |
| Brij—30 | polyoxyethylene lauryl ether | nonionic |
| Cerfak 1300 | alkyl polyoxyethylene thioether | nonionic |
| Cerfak 1400 | alkyl polyoxyethylene ether | nonionic |
| DAXAD 21 | mono-calcium salt of polymerized aryl alkyl sulfonic acids | anionic |
| Docosanol | $Ch_3(CH_2)_{21}OH$ (behenyl alcohol) | nonionic |
| Fenapon EO—526 | sodium salts of sulfated nonylphenoxypoly (ethyleneoxy) | anionic |
| Hexadecanol | $CH_3(CH_2)_{15}OH$ (behenyl alcohol) | nonionic |
| Hyamine 2389 | 40% methyl dodecyl benzyl trimethyl ammonium chloride, 10% methyl dodecyl xylylene bis trimethyl ammonium chloride, 50% HOH | cationic |
| Intexon NP—3 | nonyl phenol polyglycol ether alcohol | nonionic |
| Intexon NP—11 | nonyl phenol polyglycol ether alcohol | nonionic |
| Intexon OP—3 | octyl phenol polyglycol ether alcohol | nonionic |
| Nonic 260 | polyethylene glycol tertdodecyl thioether | nonionic |
| Plurinic F—38 | a condensate of ethylene oxide with a hydrophobic base formed by condensing propylene oxide with propylene glycol | nonionic |
| Santomerse E<br>Santomerse No. 1 | dodecylbenzene sodium sulfonate | anionic |
| Santomerse 80 | alkyl aryl sulfonate | anionic |

| Trade Name | Chemical composition | Ionogenic class |
|---|---|---|
| Soil Penetrant 3685 | polyoxyethylene ethanol | nonionic |
| Sorapon S—96 | sodium dodecylbenzene sulfonate | anionic |
| Sterox AJ | polyoxyethylene ether | nonionic |
| Sterox CD | polyoxyethylene ester | nonionic |
| Sterox DJ | alkyl phenol polyether alcohol | nonionic |
| Sterox SK<br>Sterox 6 | polyoxyethylene thioether | nonionic |
| Surfax 505 | fatty ester | nonionic |
| Surfonic LF—7 | alkyl polyoxyalkylene ether | nonionic |
| Surfonic N—95 | alkyl aryl polyethylene glycol ether | nonionic |
| Tergitol NPX | nonyl phenyl polyethylene glycol ether | nonionic |
| Tergitol TMN | trimethyl nonyl polyethylene glycol ether | nonionic |
| Tergitol 7 | sodium sulfate derivative of 3,9 diethyl tridecanol-6 | anionic |
| Tergitol 12—P—12 | dodecyl phenyl polyethylene glycol ether | nonionic |
| Tetronic 707 | compounds formed by sequential addition of propylene and ethylene oxides to ethylenediamine | nonionic |
| Tetronic 908 | (polyoxyethylene)(polyoxypropylene) ethylenediamine | nonionic |
| Triton X—100 | isoctyl phenyl polyethoxy ethanol | nonionic |
| Tween 20 | polyoxyethylene sorbitan monolaurate | nonionic |
| Tween 40 | polyoxyethylene sorbitan monopalmitate | nonionic |
| Tween 60 | polyoxyethylene sorbitan monostearate | nonionic |
| Tween 80 | polyoxyethylene sorbitan monooleate | nonionic |
| Ultrawet DS | alkyl aryl benzene aryl benzene sodium sulfonate | anionic |
| Vatsol OT | dioctyl ester of sodium sulfosuccinic acid | anionic |
| Vatsol OTB | dioctyl ester of sodium sulfosuccinic acid | anionic |
| Victawet 12 | organic phosphate ester | nonionic |

The preferred surfactant is isoctyl phenyl poly ethoxy ethanol (Triton X—100). Surfactants are used with the vehicles in ratios between 1 to 10 parts surfactant to vehicle to 10 to 1 parts surfactant to vehicle and preferably in the range of 1 to 3 parts surfactant to vehicle to 3 to 1 parts surfactant to vehicle. An especially useful composition comprises Triton X—100 and the vehicle 1-n-dodecylazacycloheptan-2-one

in a ratio between 1 to 3 and 3 to 1 parts of surfactant to vehicle and preferably 3 parts surfactant to 2 parts vehicle.

Other suitable adjuvants, surfactants and herbicides include those set forth in *Adjuvants for Herbicides,* 1982, Published by the Weed Society of America, 309, West Clark Street, Champaign, Illinois, edited by R. Hodgson.

The precise amount of the pesticide composition to be delivered to the plant or pest will obviously be an effective amount for the desired result expected therefrom. Most modern pesticides are used in agriculture at a dosage of less than 1.12 kg/ha (one pound per acre). This, of course, will be ascertained by the ordinary skill of the practitioner. Due to enhanced activity which is achieved, the dosage of agent may often be decreased from that generally applicable. In accordance with the usual prudent formulating practices, a dosage near the lower end of the useful range of the particular agent may be employed initially and the dosage increased as indicated from the observed response.

The examples which follow illustrate the vehicles which may be used in carrying out the method of the present invention. Temperatures are given in degrees Centigrade. All reactions involving sodium hydride were carried out in an inert nitrogen atmosphere.

Example 1

Preparation of 1-methylazacycloheptan-2-one having the formula

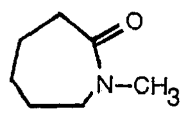

A suspension of 8.42 g of 57% sodium hydride-mineral oil suspension (4.8 g NaH, 0.2 M) was washed with 2 × 400 ml portions of dry toluene and the toluene washings were decanted. 350 ml of dry toluene was added and the suspension was mechanically stirred while a solution of 20 g (0.177 M) of azacycloheptan-2-one in 50 ml of dry toluene was added dropwise over 1 hour. After the addition was over, the mixture was refluxed for 1 hour and then cooled to room temperature. 22.0 g (0.2 M) of methyl mesylate was added dropwise over 1 hour and the reaction mixture was then warmed to 50° for 1 hour. The mixture was cooled, filtered and the filter cake was resuspended in 100 ml of dry toluene and filtered. This combined to yield 20 g (88.85%) of 1-methylazacycloheptan-2-one; b.p. 85—87°/0.13 mbar (0.1 mm. Hg).

Example 2

Preparation of 1-propylazacycloheptan-2-one having the formula

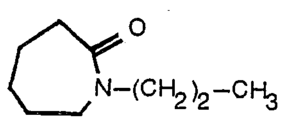

In a 1 liter 3-neck flask equipped with a dry ice/isopropanol condenser, an addition funnel and a mechanical stirrer was placed 10.2 g of 50% sodium hydride-mineral oil dispersion (5.1 g NaH, 0.2125 M) and 150 ml of petroleum ether. The suspension was momentarily stirred and then sodium hydride was allowed to settle. Most of the petroleum ether was pipetted out and 200 ml of dry toluene was added. To this was added dropwise a solution of 20 g (0.177 M) of azacycloheptan-2-one in 100 ml of dry toluene. The mixture was refluxed for 1 hour and then cooled to room temperature. A solution of 30.75 g (0.25 M) of 1-bromopropane in 100 ml of dry toluene was added dropwise under stirring. Upon completion of the addition, the mixture was warmed to 80—100° and the temperature and maintained there for 4 hours. Then the isopropanol-dry ice condenser was replaced with a water condenser and the reaction mixture was heated to reflux for 15 hours. The reaction mixture was cooled, filtered and the filtrate was concentrated to a yellow oil. Distillation afforded 22.2 g (81%) of colorless product; b.p. 83—86°/0.33 mbar (0.25 mm Hg).

Example 3

Preparation of 1-n-butylazacycloheptan-2-one having the formula

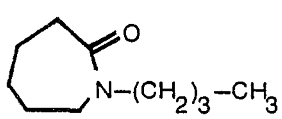

Following Example 2, from 12.75 g of 50% sodium hydride-mineral oil dispersion (6.375 g NaH, 0.266 M), 25 g (0.221 M) of azacycloheptan-2-one and 34.25 g (0.25 M) of 1-bromobutane was obtained on 18 hour reflux 26.8 g (72%) of colorless product; b.p. 95—100°/0.4 mbar (0.3 mm Hg).

Example 4

Preparation of 1-n-pentylazacycloheptan-2-one having the formula

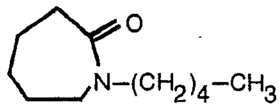

Following Example 2 and using water condenser from the start of the reaction, 10 g of 50% sodium hydride-mineral oil dispersion (5 g NaH, 0.21 M), 20 g (0.177 M) of azacycloheptan-2-one and 30.2 g (0.2 M) of 1-bromopentane on 18 hour reflux gave 23.3 g (87%) of colorless product; b.p. 110—115°/0.4 mbar (0.3 mm Hg).

Example 5

Preparation of 1-n-hexylazacycloheptan-2-one having the formula

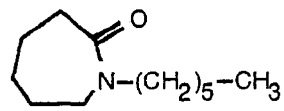

Following Example 4, from 10.2 g of 50% sodium hydride-mineral oil dispersion (5.1 g NaH, 0.2125 M), 20 g (0.177 M) of azacycloheptan-2-one and 33 g (0.2 M) of 1-bromohexane on 19 hour reflux was obtained 29.8 g (85.3%) of colorless product; b.p. 122—128°/0.53 mbar (0.4 mm Hg).

Example 6

Preparation of 1-n-heptylazacycloheptan-2-one having the formula

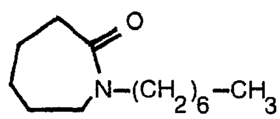

Following Example 4, 10.2 g of 50% sodium hydride-mineral oil dispersion (5.1 g of NaH, 0.2125 M), 20 g (0.177 M) of azacycloheptan-2-one and 35.8 g (0.2 M) of 1-bromoheptan on 18 hour reflux gave 33.5 g (90%) of colorless product; b.p. 155—158°/0.67 mbar (0.5 mm Hg).

Example 7

Preparation of 1-n-octylazacycloheptan-2-one having the formula

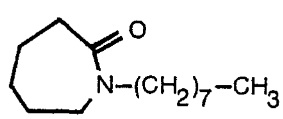

17.5 g (0.153 M) of 6-hexanolactone and 22 g (0.17 M) of 1-aminooctane were mixed and heated to 180° in a round bottom flask equipped with a condenser and a Dean-Stark-trap for 29 hours. The reaction mixture was distilled at reduced pressure to yield 8.8 g (27%) of product; b.p. 155—160°/0.67 mbar (0.5 mm Hg).

Example 8

Preparation of 1-n-nonylazacycloheptan-2-one having the formula

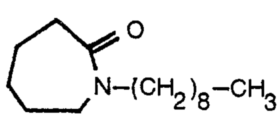

Following Example 7, heating 22.83 g (0.2 M) of 6-hexanolactone and 28.65 g (0.2 M) of 1-aminononane at 180° for 20 hours gave 11.5 g (26%) of product; b.p. 155—165°/0.8 mbar (0.6 mm Hg).

(Higher yields of 1-n-octyl- and 1-n-nonylazacycloheptan-2-one may be obtained by use of the sodium hydride method or by carrying out the reaction in a stainless bomb under pressure.)

Example 9

Preparation of 1-n-decylazacycloheptan-2-one having the formula

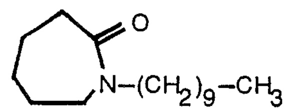

Following Example 4, 10.2 g of 50% sodium hydride-mineral oil dispersion (5.1 g NaH, 0.2125 M), 20 g (0.177 M) of azacycloheptan-2-one and 44.2 g (0.2 M) of 1-bromodecane on 19 hours reflux gave 38 g (84.7%) of product; b.p. 158—163°/0.33—0.4 m bar (0.25—0.3 mm Hg).

Example 10

Preparation of 1-n-dodecylazacycloheptan-2-one having the formula

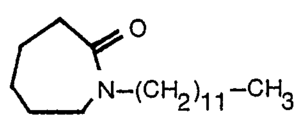

Following Example 4, 15.3 g of 50% sodium hydride-mineral oil dispersion (7.65 g NaH, 0.319 M), 30 g (0.266 M) of azacycloheptan-2-one and 66.1 g (0.265 M) of 1-bromododecane on 20 hours reflux gave 60 g (80%) of colorless product; b.p. 175—180°/0.4 mbar (0.3 mm Hg).

Example 11

Preparation of 1-n-butylazacyclononan-2-one having the formula

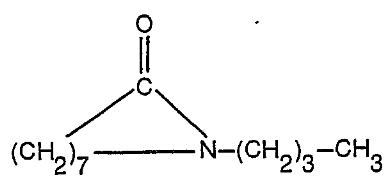

Following Example 4, 16.32 g of 50% sodium hydride-mineral oil dispersion (8.16 g NaH, 0.34 M), 40 g (0.283 M) of azacyclononan-2-one and 43 g (0.311 M) of 1-bromo-butane was refluxed for 22 hours. The reaction mixture was diluted with benzene-toluene and was extracted with water. The organic phase was separated, dried and concentrated to a yellow oil. Distillation afforded 41.4 g (74%) of product; b.p. 166—170°/0.27 mbar (0.2 mm Hg).

Example 12

Preparation of 1-n-octylazacyclononan-2-one having the formula

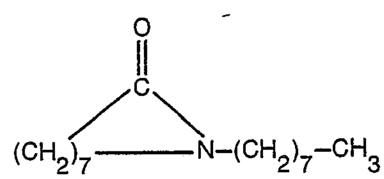

Following Example 11, 4.2 g of 50% sodium hydride-mineral oil dispersion (2.1 g NaH, 0.0875 M), 10 g (0.0708 M) of azacyclononan-2-one and 15 g (0.0777 M) of 1-bromooctane gave 12.5 g (70%) of product; b.p. 150—160°/0.67 mbar (0.5 mm Hg).

Example 13

The herbicidal activity of a commercially available pesticide, glyphosate (Monsanto Chemical Company—Roundup®) in combination with varying concentrations of the vehicle 1-n-dodecyl-azacycloheptan-2-one was tested on two common weeds Hybrid Bermuda grass (Cymodon Doctylon) and St. Augustine's grass (Stenotaphrum Secundatum). The glyphosat was tested at the midrange of the manufacturer's recommended spray solution concentration of 1.5% and at ¼ of this concentration or ⅜%. The vehicle was used at four concentrations of 0.1%, 1.0%, 2.0% and 5%. The test solutions were made up with the indicated concentrations of glyphosate and vehicle in aqueous compositions containing 18% ethanol and 6% Tween 20 and applied in a fine mist with a delivery of 0.3 g of solution made to each 4.4 square inch test site. Thereafter, the test sites were watered for 20 minutes each 48 hours for 17 days. The results of the 17 day study showed that the vehicle effectively enhanced the herbicidal activity of

glyphosate at a concentration of 1.5% substantially reducing kill time compared to 1.5% glyphosate alone. The studies further demonstrated that the vehicle also potentiated the herbicidal effect of glyphosate at $\frac{1}{4}$ of the recommended dilution of $\frac{3}{8}$%. The best results were achieved with vehicle concentrations of 1% or higher.

**Claims**

1. A method for improved plant pest control comprising contacting a plant or plant pest with a composition comprising an effective amount of a plant pesticide and 0.1 to 10% by weight of the pesticide composition of a compound having the structural formula:

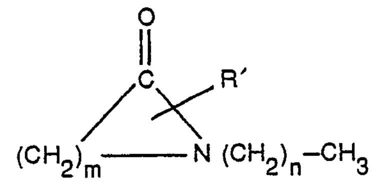

wherein R′ is H of alkyl having 1—4 carbon atoms, m is 5—7 and n is 0—11.

2. The method of claim 1 wherein in the formula R′ is hydrogen, m is 5, n is 6 to 9 or 11, or wherein R′ is hydrogen, m is 7 and n is 3 or 7.

3. The method of claim 1 wherein the plant pesticide is an insecticide.

4. The method of claim 1 wherein the plant pesticide is a fungicide.

5. The method of claim 1 wherein the plant pesticide is a herbicide.

6. The method of claim 1 wherein the plant pesticide is a rodenticide.

7. The method of claim 1 wherein the plant pesticide is a nematicide.

8. The method of claim 1 wherein the plant pesticide is a molluscicide.

9. The method of claim 1 wherein the plant pesticide is an acaricide.

10. A method of claim 1 for improved plant pest control comprising contacting a plant or plant pest with a composition comprising an effective amount of a plant pesticide and 0.1 to 10% by weight of the pesticidal composition of 1-n-dodecylazacycloheptan-2-one.

11. The method of claim 10 wherein the plant pesticide is an insecticide.

12. The method of claim 10 wherein the plant pesticide is a fungicide.

13. The method of claim 10 wherein the plant pesticide is a herbicide.

14. The method of claim 10 wherein the plant pesticide is a rodenticide.

15. The method of claim 10 wherein the plant pesticide is a nematicide.

16. The method of claim 10 wherein the plant pesticide is an molluscicide.

17. The method of claim 10 wherein the plant pesticide is an acaricide.

18. A pesticide composition comprising a surfactant suitable for use as a herbicide adjuvant and 0.1 to 10% by weight of the pesticide composition of a compound having the structural formula

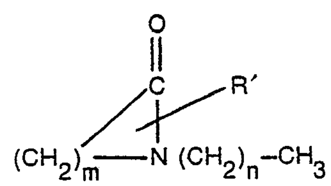

wherein R′ is H or alkyl having 1—4 carbon atoms, m is 5—7 and n 0—11.

19. The composition of claim 18 wherein in the formula R′ is hydrogen, m is 5, n is 6 to 9 or 11, or wherein R′ is hydrogen, m is 7 and n is 3 or 7.

20. The composition of claim 18 comprising a surfactant suitable for use as a herbicide adjuvant and 0.1 to 10% by weight of the pesticide composition of 1-n-dodecylazacycloheptan-2-one.

21. The composition of claim 20 wherein the surfactant is isoctyl phenyl polyethoxy ethanol.

22. The composition of claim 21 wherein the ratio of surfactant to compound is between 3 to 1 and 1 to 3 parts by weight surfactant to compound.

23. The composition comprising an effective amount of a plant pesticide and 0.1 to 10% by weight of the pesticide composition of a compound having the structural formula

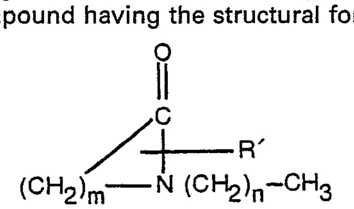

wherein R′ is H or alkyl having 1—4 carbon atoms, m is 5—7 and n is 0—11.

24. The composition of claim 23 wherein in the formula R′ is hydrogen, m is 5, n is 6 to 9 or 11, or wherein R′ is hydrogen, m is 7 and n is 3 or 7.

25. The composition of claim 23 comprising an effective amount of a plant pesticide and 0.1 to 10% by weight of the pesticidal composition of 1-n-dodecylazacycloheptan-2-one.

**Patentansprüche**

1. Verfahren zur Verbesserung der Kontrolle von Pflanzenschädlingen, dadurch gekennzeichnet, daß man die Pflanze oder den Pflanzenschädling mit einem Mittel behandelt, welches eine wirksame Menge eines Pflanzenpestizids und 0,1 bis 10 Gew.-%, bezogen auf die Pestizidzusammensetzung, einer Verbindung der Strukturformel:

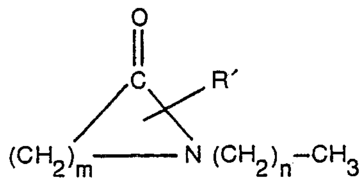

worin R′ H oder Alkyl mit 1 bis 4 Kohlenstoffatomen bedeutet, m 5 bis 7 und n 0 bis 11 bedeuten, enthält.

2. Verfahren nach Anspruch 1, dadurch gekennzeichnet, daß in der Formel R′ Wasserstoff, m 5, n 6 bis 9 oder 11 bedeuten, oder daß R′ Wasserstoff, m 7 und n 3 oder 7 bedeuten.

3. Verfahren nach Anspruch 1, dadurch gekennzeichnet, daß das Pflanzenpestizid ein Insektizid ist.

4. Verfahren nach Anspruch 1, dadurch gekennzeichnet, daß das Pflanzenpestizid ein Fungizid ist.

5. Verfahren nach Anspruch 1, dadurch gekennzeichnet, daß das Pflanzenpestizid ein Herbizid ist.

6. Verfahren nach Anspruch 1, dadurch gekennzeichnet, daß das Pflanzenpestizid ein Rodentizid.

7. Verfahren nach Anspruch 1, dadurch gekennzeichnet, daß das Pflanzenpestizid ein Nematizid ist.

8. Verfahren nach Anspruch 1, dadurch gekennzeichnet, daß das Pflanzenpestizid ein Molluskizid ist.

9. Verfahren nach Anspruch 1, dadurch gekennzeichnet, daß das Pflanzenpestizid ein Akarizid ist.

10. Verfahren nach Anspruch 1 für eine verbesserte Kontrolle von Pflanzenschädlingen, dadurch gekennzeichnet, daß man die Pflanze oder den Pflanzenschädling mit einer Zusammensetzung behandelt, welche eine wirksame Menge eines Pflanzenpestizids und 0,1 bis 10 Gew.-%, bezogen auf die Pestizidzusammensetzung, von 1-n-Dodecylazacycloheptan-2-on enthält.

11. Verfahren nach Anspruch 10, dadurch gekennzeichnet, daß das Pflanzenpestizid ein Insektizid ist.

12. Verfahren nach Anspruch 10, dadurch gekennzeichnet, daß das Pflanzenpestizid ein Fungizid ist.

13. Verfahren nach Anspruch 10, dadurch gekennzeichnet, daß das Pflanzenpestizid ein Herbizid ist.

14. Verfahren nach Anspruch 10, dadurch gekennzeichnet, daß das Pflanzenpestizid ein Rodentizid ist.

15. Verfahren nach Anspruch 10, dadurch gekennzeichnet, daß das Pflanzenpestizid ein Nematizid ist.

16. Verfahren nach Anspruch 10, dadurch gekennzeichnet, daß das Pflanzenpestizid ein Molluskizid ist.

17. Verfahren nach Anspruch 10, dadurch gekennzeichnet, daß das Pflanzenpestizid ein Akarizid ist.

18. Pestizidzusammensetzung, dadurch gekennzeichnet, daß sie ein oberflächenaktives Mittel, das als Herbizidadjuvans geeignet ist und 0,1 bis 10 Gew.-%, bezogen auf die Pestizidzusammensetzung, einer Verbindung der Strukturformel:

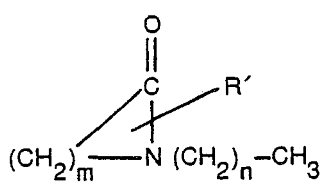

worin R′ H oder Alkyl mit 1 bis 4 Kohlenstoffatomen, m 5 bis 7 und n 0 bis 11 bedeuten, enthält.

19. Zusammensetzung nach Anspruch 18, dadurch gekennzeichnet, daß in der Formel R′ Wasserstoff, m 5, n 6 bis 9 oder 11 bedeuten, oder daß R′ Wasserstoff, m 7 und n 3 oder 7 bedeuten.

20 Zusammensetzung nach Anspruch 18, dadurch gekennzeichnet, daß sie ein oberflächenaktives Mittel, das als Herbizidadjuvans geeignet ist, und 0,1 bis 10 Gew.-%, bezogen auf die Pestizidzusammensetzung, von 1-n-Dodecylazacycloheptan-2-on enthält.

21. Zusammensetzung nach Anspruch 20, dadurch gekennzeichnet, daß das oberflächenaktive Mittel Isoctylphenylpolyethoxyethanol ist.

22, Zusammensetzung nach Anspruch 21, dadurch gekennzeichnet, daß das Verhältnis des oberflächenaktiven Mittels zu der Verbindungen zwischen 3 bis 1 und 1 bis 3 Gew.-Teilen oberflächenaktives Mittel zu der Verbindung liegt.

23. Zusammensetzung, dadurch gekennzeichnet, daß sie eine wirksame Menge eines Pflanzenpestizids und 0,1 bis 10 Gew.-%, auf die Pestizidzusammensetzung, einer Verbindung der Strukturformel:

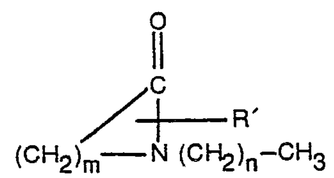

worin R' Wasserstoff oder Alkyl mit 1 bis 4 Kohlenstoffatomen, m 5 bis 7 und n 0 bis 11 bedeuten, enthält.

24. Zusammensetzung nach Anspruch 23 daduch gekennzeichnet, daß in der Formel R' Wasserstoff, m 5, n 6 bis 9 oder 11 bedeuten, oder daß R' Wasserstoff, m 7 und n 3 oder 7 bedeuten.

25. Zusammensetzung nach Anspruch 23, dadurch gekennzeichnet, daß sie eine wirksame Menge eines Pflanzenpestizids und 0,1 bis 10 Gew.-%, bezogen auf die Pestizidzusammensetzung, von 1-n-Dodecylazacycloheptan-2-on enthält.

**Revendications**

1. Procédé phytosanitaire perfectionné comprenant la mise en contact d'une plante ou d'un nuisible pour les végétaux avec une composition comprenant une quantité efficace d'un pesticide pour végétaux et 0,1 à 10% en poids de la composition pesticide d'un composé ayant la formule structurale

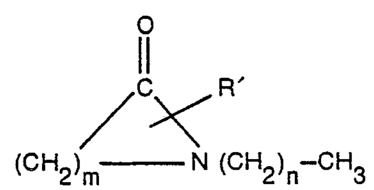

dans laquelle R' est H ou un groupe alcoyl ayant 1 à 4 atomes de carbone, m est 5 à 7 et n est 0 à 11.

2. Procédé suivant la revendication 1, où, dans la formule R' est un atome d'hydrogène, m est 5, n est 6 à 9 ou 11, ou bien, dans laquelle R' est un atome d'hydrogène, m est 7, et n est 3 ou 7.

3. Procédé suivant la revendication 1, dans lequel le pesticide est un insecticide.

4. Procédé suivant la revendication 1, dans lequel le pesticide est un fongicide.

5. Procédé suivant la revendication 1, dans lequel le pesticide est un herbicide.

6. Procédé suivant la revendication 1, dans lequel le pesticide est un rodenticide.

7. Procédé suivant la revendication 1, dans lequel le pesticide est un nématicide.

8. Procédé suivant la revendication 1, dans lequel le pesticide détruit les mollusques.

9. Procédé suivant la revendication 1, dans lequel le pesticide est un acaricide.

10. Procédé phytosanitaire perfectionné suivant la revendication 1 comprenant la mise en contact d'une plante ou d'un nuisible pour végétaux, avec une composition comprenant une quantité efficace d'un pesticide et 0, 1 à 10% en poids de la composition pesticide de:

1-n-dodécylazacycloheptan-2-one.

11. Procédé suivant la revendication 10, dans lequel le pesticide est un insecticide.

12. Procédé suivant la revendication 10, dans lequel le pesticide est un fongicide.

13. Procédé suivant la revendication 10, dans lequel le pesticide est un herbicide.

14. Procédé suivant la revendication 10, dans lequel le pesticide est un rodenticide.

15. Procédé suivant la revendication 10, dans lequel le pesticide est un nématicide.

16. Procédé suivant la revendication 10, dans lequel le pesticide détruit les mollusques.

17. Procédé suivant la revendication 10, dans lequel le pesticide est un acaricide.

18. Composition pesticide comprenant un tensioactif pouvant être utilisé comme adjuvant herbicide et 0,1 à 10% en poids de la composition pesticide d'un composé ayant la formule structurale

18. Composition pesticide comprenant un tensio-actif pouvnt être utilisé comme adjuvant herbicide et 0,1 à 10% en poids de la composition pesticide d'un composéayant la formule structurale

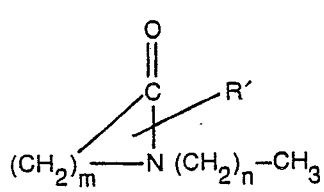

dans laquelle R' est H ou un groupe alcoyle ayant 1 à 4, atomes de carbone, m est 5—7 et n est 0—11.

19. Composition suivant la revendication 18, où, dans la formule, R′ est un atome d'hydrogène, m est 5, n est 6 à 9 ou 11, ou bien dans laquelle R′ est un atome d'hydrogène, m est 7 et n est 3 ou 7.

20. Composition suivant la revendication 18, comprenant un tensioactif pouvant être utilisé comme adjuvant herbicide et 0,1 à 10% en poids de la composition pesticide de 1-n-dodécylazacycloheptan-2-one.

21. Composition suivant la revendication 20, dans laquelle le tensioactif est l'isoctyl phényl polyéthoxy éthanol.

22. Composition suivant la revendication 21, dans laquelle le rapport du tensioactif au composé est entre 3 à 1 et 1 à 3 parties en poids de tensioactif au composé.

23. Composition comprenant une quantité efficace d'un pesticide pour végétaux et 0,1 à 10% en poids de la composition pesticide d'un composé ayant la formule structurale:

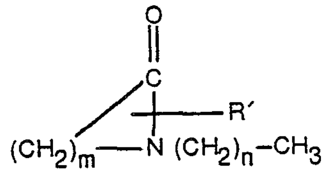

dans laquelle R′ est H ou un groupe alcoyle ayant 1 à 4 atomes de carbone, m est 5—7 et n est 0—11.

24. Composition suivant la revendication 23 dans laquelle dans la formule, R′ est un atome d'hydrogène, m est 5, n est 6 à 9 ou 11, ou bien R′ est un atome d'hydrogène, m est 7 et N est 3 ou 7.

25. Composition suivant la revendication 23 comprenant une quantité efficace d'un pesticide pour végétaux et 0,1 à 10% en poids de la composition pesticide de 1-n-dodécylazacycloheptan-2-one.